# EUROPEAN PATENT APPLICATION

(11) **EP 4 706 540 A1**
(43) Date of publication of application: **11.03.2026**
(21) Application number: 24814962.7
(22) Date of filing: 02.04.2024
(51) Int. Cl.: A61B 5/372, A61B 5/291, A61B 5/369, A61B 5/00, A61B 5/374

(54) **BRAIN WAVE CORRECTION DEVICE AND BRAIN WAVE CORRECTION METHOD**

(30) Priority: 31.05.2023 JP 2023089548
(71) Applicant: JVCKenwood Corporation, Yokohama-shi, Kanagawa 2210022 (JP)
(72) Inventor: MARUYAMA, Toshihiro, Yokohama-shi, Kanagawa 221-0022 (JP)
(74) Representative: Wagner & Geyer
(86) International application number: PCT/JP2024/013614
(87) International publication number: WO 2024/247475

(57) **Abstract**

At a body part other than the head region, brain wave signals having high accuracy are obtained. A brain wave correction device includes: a transfer function obtaining unit (411) that, based on a brain wave signal at a first body part and a brain wave signal at a second body part different than the first body part at the time when a user (SU) has a predetermined thought (IM), obtains a transfer function for brain wave signals between the body parts; a brain wave obtaining unit (412) that obtains a brain wave signal at the second body part; a brain wave correcting unit (413) that corrects, based on the transfer function, the brain wave signal obtained at the second body part and calculates a corrected head-region brain wave signal; and a brain wave comparing unit (414) that, based on the corrected head-region brain wave signal and based on a reference brain wave signal obtained in advance at the first body part, estimates the thought arising in the user (SU).

## Description

### Field

The application concerned relates to a brain wave correction device and a brain wave correction method. Background

The research has been going on about a method for obtaining the brain wave signals of a person in an accurate manner. In Patent Literature 1, a brain wave measurement device is disclosed in which a plurality of sensors to be attached to the head region is disposed in a unit capable of controlling the amount of contact and the contact pressure with respect to the head region.

### Citation List

### Patent Literature

[Patent Literature 1] Japanese Patent Application Laid-open No. 2022-033419

### Summary

### Technical Problem

At the time of utilizing a brain wave sensor in daily life, attaching the brain wave sensor to the head region is a very demanding task for the user. On the other hand, when a sensor is attached to a body part separated from the head region, the obtained brain wave signals undergo attenuation, and there is a significant decline in the accuracy as compared to the measurement result obtained at the head region. Hence, there is a demand for being able to accurately obtain the brain wave signals at a body part other than the head region.

Embodiments described below are in view of the issues mentioned above, and it is an objective to provide a brain wave correction device, a brain wave correction method, and a program that enable obtaining brain wave signals having high accuracy.

### Solution to Problem

A brain wave correction device according to the present disclosure comprising: a transfer function obtaining unit that, based on a brain wave signal at a first body part and a brain wave signal at a second body part different than the first body part at time when a user has a predetermined thought, obtains a transfer function for brain wave signals between the body parts; a brain wave obtaining unit that obtains a brain wave signal at the second body part; a brain wave correcting unit that calculates a corrected head-region brain wave signal which is a brain wave signal obtained by correcting, based on the transfer function, the brain wave signal obtained at the second body part; and a brain wave comparing unit that, based on the corrected head-region brain wave signal and based on a reference brain wave signal obtained in advance at the first body part, estimates thought arising in the user.

A brain wave correction method according to the present disclosure comprising: an obtaining step that, based on a brain wave signal at a first body part and a brain wave signal at a second body part different than the first body part at time when a user has a predetermined thought, includes obtaining a transfer function for brain wave signals between the body parts; an obtaining step for obtaining a brain wave signal at the second body part; a calculating step that includes correcting, based on the transfer function, the brain wave signal obtained at the second body part and calculating a corrected head-region brain wave signal; and an estimating step that, based on the corrected head-region brain wave signal and based on a reference brain wave signal obtained in advance at the first body part, includes estimating thought arising in the user.

According to the present embodiments, it becomes possible to provide a brain wave correction device and a brain wave correction method that enable obtaining brain wave signals having high accuracy.

### Brief Description of Drawings

FIG. 1 is a schematic diagram of a brain wave sensor system according to a first embodiment.
FIG. 2 is a schematic block diagram of a brain wave correction device according to the first embodiment.
FIG. 3 is a schematic diagram for explaining the operations performed by a transfer function obtaining unit according to the first embodiment.
FIG. 4 is an explanatory diagram regarding an estimation operation performed for estimating the thought of the user from the brain wave signal obtained at a second body part.
FIG. 5 is a flowchart for explaining the flow of operations performed in the brain wave correction device according to the first embodiment.
FIG. 6 is a schematic diagram of the brain wave measurement device according to a second embodiment. Description of Embodiments

Preferred embodiments of the application concerned are described below in detail with reference to the accompanying drawings. However, the application concerned is not limited by the embodiments described below. Moreover, in the case of having a plurality of embodiments, combinations thereof are also considered applicable.

### (First embodiment)

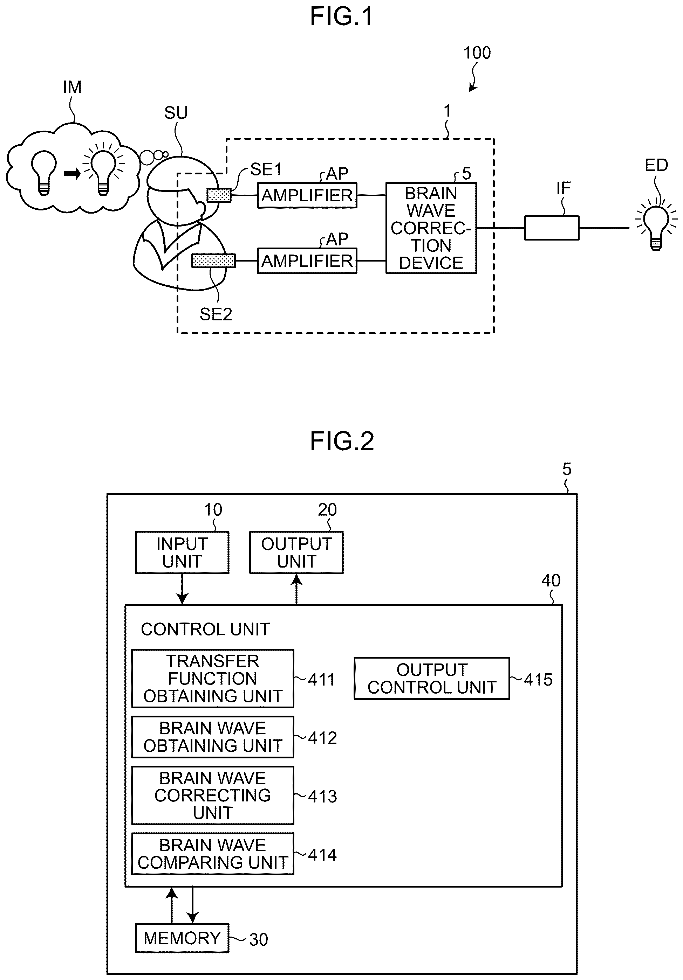

Explained below with reference to FIG. 1 is a configuration of a brain wave sensor system. FIG. 1 is a schematic diagram of a brain wave sensor system according to a first embodiment.

### (Brain wave sensor system)

A brain wave sensor system 100 illustrated in FIG. 1 according to the first embodiment is capable of controlling the operations of an external device ED based on the brain wave signals generated at the time when a user SU has a predetermined thought IM. In the first embodiment, a brain wave measurement device 1 is capable of: detecting the brain wave signals generated at the time when the user SU has the predetermined thought IM about turning on the light bulb representing the external device ED; outputting, to the light bulb representing the external device ED, a control signal for turning on the light bulb; and accordingly turning on the light bulb representing the external device ED. Thus, in the first embodiment, a light bulb represents the external device ED, and turning on the light bulb represents the predetermined thought IM. However, the external device ED is not limited to be a light bulb, and can be an arbitrary device. Moreover, the predetermined thought IM can be an arbitrary thought. However, it is desirable that the predetermined thought IM is about operating the external device ED.

As illustrated in FIG. 1, the brain wave sensor system 100 includes the brain wave measurement device 1, an external device interface 1F, and the external device ED. The external device ED represents the target controlled by the user SU with the use of the brain waves. The external device ED is connected to an external device interface IF. The external device interface IF is a device that connects the brain wave measurement device 1 to the external device ED, that receives a control signal from the brain wave measurement device 1, and that converts the control signal into an instruction signal for operating the external device ED. The external device interface IF either can be included inside the external device ID for implementing the functions explained above, or can be configured using different hardware than the external device ED. Meanwhile, the external device interface IF can be configured to include an MCU (Micro Controller Unit) so as to enable more sophisticated control of the external device ED.

The brain wave measurement device 1 is a device that obtains brain wave signals of the user SU and, when it is estimated that the obtained brain wave signals are generated when the user SU has the predetermined through IM (the thought about operating the external device ED), outputs a control signal for operating the external device ED. The brain wave measurement device 1 includes a brain wave correction device 5; a first sensor SE1; a second sensor SE2; and amplifiers AP one of which is connected between the first sensor SE1 and the brain wave correction device 5 and the other is connected between the second sensor SE2 and the brain wave correction device 5.

The first sensor SE1 and the second sensor SE2 are sensors for obtaining the brain wave signals of the user SU. More particularly, the first sensor SE1 and the second sensor SE2 can be EEGs (Electroencephalography) that make use of electrodes to detect the brain waves from the weak current flowing in the neural network of the brain. For example, the first sensor SE1 and the second sensor SE2 can be implemented using the sensor type in which a conducting gel is applied in between the skin of the user SU and the electrode so that the contact resistance between the electrode and the skin is lowered, or the sensory type in which a sponge soaked in an electrolyte solution is placed in between the skin and the electrode. The first sensor SE1 and the second sensor SE2 obtain brain wave signals (electrical signals) from the weak current based on the thoughts arising in the user SU. The first sensor SE1 according to the first embodiment is attached to the head region representing a first body part, and the second sensor SE2 is attached to a second body part that is different than the first body part. That is, the second body part, to which the second sensor SE is attached, indicates a body part other than the head region of the user SU. As an example according to the first embodiment, the second body part indicates an arm of the user SU. However, the second body part can be an arbitrary body part other than the head region of the user SU. Meanwhile, the brain wave signals detected by the first sensor SE1 and the second sensor SE2 can be, for example, electrical signals generated from the user SU and having the frequency in the range between 0 Hz or higher and 30 Hz or lower.

The amplifiers AP are amplifiers that allow passage of only those brain wave signals which are present within the brain wave signal bandwidth representing a predetermined frequency band, and amplify the brain wave signals that have passed through. The brain wave signal bandwidth can be an arbitrary frequency band and, for example, can be equal to or lower than 1 KHz. As an example of a specific brain wave bandwidth, α waves can be in the range between 8 Hz or higher and 13 Hz or lower; β waves can be in the range between 14 Hz or higher and 30 Hz or lower; and γ waves can be in the range between 30 Hz or higher and 64 Hz or lower. Meanwhile, the brain wave signals, which have passed through the amplifiers AP, can be amplified by an arbitrary degree of amplification such as about 40 db. From among the signals obtained from the first sensor SE1 and the second sensor SE2, the signals present outside the brain wave signal bandwidth are blocked as noise by the amplifiers AP, and the signals that are present within the brain wave signal bandwidth and that have passed through the amplifiers AP are amplified by the amplifiers AP and are then input to the brain wave correction device 5.

### (Brain wave correction device)

FIG. 2 is a schematic block diagram of a brain wave correction device according to the first embodiment. The brain wave correction device 5 is a device that obtains brain wave signals using a first sensor and a second sensor; estimates the thought of the user SU based on the obtained brain wave signals; and outputs a control signal for controlling the external device ED corresponding to the estimated thought of the user SU. The brain wave correction device 5 according to the first embodiment is, for example, a computer and, as illustrated in FIG. 2, includes an input unit 10, an output unit 20, a memory 30, and a control unit 40. The input unit 10 is a device that receives an operation performed by the user; and, for example, can be a mouse, a keyboard, or a touch-sensitive panel. The output unit 20 is a device that outputs information; and, for example, can be an output terminal that outputs a control signal for the external device ED.

The memory 30 is a memory for storing the details of arithmetic processing performed by the control unit 40, for storing programs, and for storing a variety of information input by the user SU using the input unit 10. For example, the memory 30 includes at least either a main storage device such as a RAM (Random Access Memory) or a ROM (Read Only Memory), or an external storage device such as an HDD (Hard Disk Drive). The program that is stored in the memory 30 for use by the control unit 40 can alternatively be stored in a recording medium readable by the brain wave correction device 5.

The control unit 40 is an arithmetic device that includes an arithmetic circuit such as a CPU (Central Processing Unit). The control unit 40 includes a transfer function obtaining unit 411, a brain wave obtaining unit 412, a brain wave correcting unit 413, a brain wave comparing unit 414, and an output control unit 415. The control unit 40 reads the program (software) from the memory 30 and executes it so as to implement the transfer function obtaining unit 411, the brain wave obtaining unit 412, the brain wave correcting unit 413, the brain wave comparing unit 414, and the output control unit 415; and performs the corresponding operations. The control unit 40 either can perform the operations using a single CPU or can include a plurality of CPU and perform the operations using those CPUs. Meanwhile, at least some of the transfer function obtaining unit 411, the brain wave obtaining unit 412, the brain wave correcting unit 413, the brain wave comparing unit 414, and the output control unit 415 can be implemented using hardware.

### (Operations performed by brain wave correction device)

Given below is the explanation of the details of the operations performed by the brain wave correction device 5.

### (Transfer function)

The brain wave correction device 5 is capable of obtaining high-accuracy brain wave signals at a body part other than the head region. More specifically, regarding the brain wave signals that are commonly generated in the brain of the user SU, as the distance from the head region goes on increasing, there is attenuation of the brain wave signals in accordance with a transfer function. Hence, when the brain wave signals are obtained at a body part separated from the head region, there is a drop in the accuracy of the brain wave signals. Regarding the brain wave signals obtained at the first body part and the brain wave signals obtained at the second body part, the brain wave correction device 5 calculates a transfer function for brain wave signals between body parts; and, using the calculated transfer function, can calculate the brain wave signals at the first body part from the brain wave signals at the second body part. That is, the transfer function is a function for converting the brain wave signals at the second body part into the brain wave signals at the first body part. In other words, as a result of using the transfer function, the brain wave correction device 5 can infer the high-accuracy brain wave signals at the first body part from the attenuated and low-accuracy brain wave signals at the second body part. Based on the brain wave signals at the first body part and the brain wave signals at the second body part, the brain wave correction device 5 according to the first embodiment calculates, in advance, the transfer function for brain wave signals between body parts; uses the transfer function for correction (conversion) of the brain wave signals at the second body part; and infers the brain wave signals at the first body part. Given below is the explanation of a method for calculating the transfer function according to the first embodiment.

### (Calculation of transfer function)

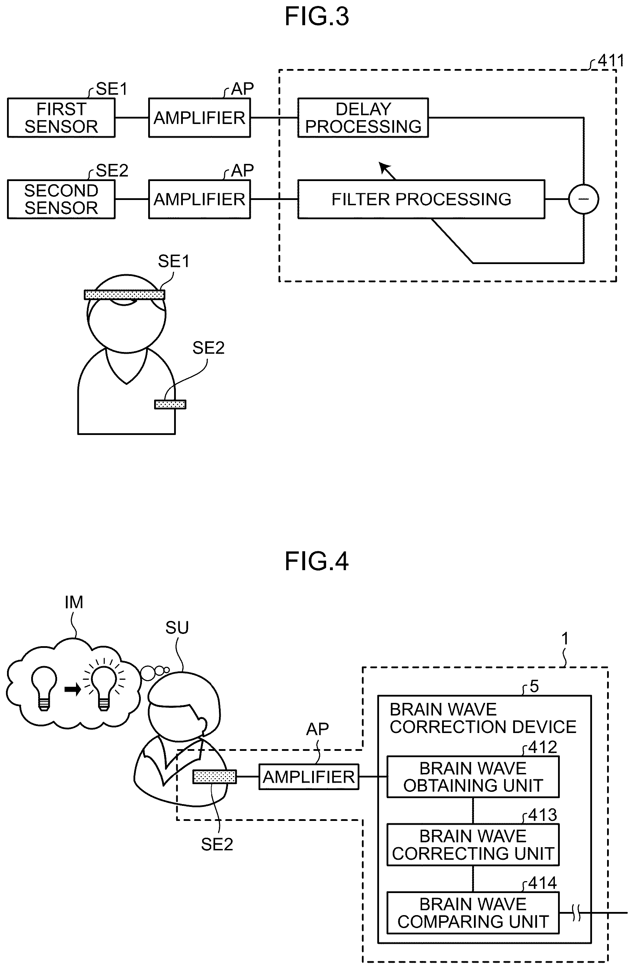

FIG. 3 is a schematic diagram for explaining the operations performed by the transfer function obtaining unit according to the first embodiment. The transfer function obtaining unit 411 obtains a transfer function for brain wave signals between the first body part and the second body part based on the brain wave signal obtained at the first body part and the brain wave signal obtained at the second body part. More particularly, using the first sensor SE1 attached to the first body part and using the second sensor SE2 attached to the second body part, the transfer function obtaining unit 411 obtains the brain wave signals generated when the predetermined thought IM arises in the user SU; and calculates a transfer function based on each brain wave signal. The predetermined thought IM at that time can be set in an arbitrary manner. For example, the predetermined thought IM can be about operating the external device ED, or can be a thought unrelated to the operation of the external device ED.

Of the brain wave signals obtained by the transfer function obtaining unit 411 from the first body part and from the second body part, the signals present outside the brain wave signal bandwidth are cut off as noise by the amplifiers AP and only the signals present within the brain wave signal bandwidth are amplified by the amplifiers AP and are used in calculating the transfer function. In the following explanation, the brain wave signal that is obtained using the first sensor SE1 and that is amplified by the corresponding amplifier AP is called a head-region brain wave signal, and the brain wave signal that is obtained using the second sensor SE2 and that is amplified by the corresponding amplifier AP is called an arm-region brain wave signal.

### (Delay processing)

The transfer function obtaining unit 411 calculates the transfer function based on a head-region brain wave signal and an arm-region brain wave signal. More particularly, the transfer function obtaining unit 411 calculates the transfer function based on the difference between a head-region brain wave signal that is obtained and an arm-region brain wave signal that has been subjected to filter processing (explained later). At that time, before obtaining the difference, it is desirable that delay processing is performed with respect to the head-region brain wave signal. Usually, since a brain wave signal is generated inside the brain, the timing at which the brain wave signal reaches the second body part has a time difference (delay) with respect to the timing at which the brain wave signal reaches the first body part. For that reason, in that regard, the transfer function obtaining unit 411 performs delay processing with respect to the head-region brain wave signal, so that the timing of obtaining the head-region brain wave signal matches with the timing of obtaining the arm-region brain wave signal. In other words, the transfer function obtaining unit 411 calculates the delay period of the brain wave signal obtained at the second body part (i.e., the arm-region brain wave signal) with respect to the brain wave signal at the first body part (i.e., the head-region brain wave signal); corrects the brain wave signal at the first body part (i.e., the head-region brain wave signal) based on the delay period; obtains, based on the post-correction brain wave signal at the first body part (i.e., the head-region brain wave signal) and the brain wave signal at the second body part (i.e., the arm-region brain wave signal) that has been subjected to filter processing, the difference between the two brain wave signals; and calculates the transfer function for brain wave signals between the first body part and the second body part. Given below is the explanation about the filter processing.

### (Filter processing)

In FIG. 3 are schematically illustrated the details of the operation by which the transfer function obtaining unit 411 obtains the difference between the head-region brain wave signal, which has been subjected to delay processing, and the arm-region brain wave signal, which has been subjected to filter processing; and updates the filter factor in the filter processing based on the obtained difference. More particularly, the transfer function obtaining unit 411 obtains the difference between the head-region brain wave signal, which has been subjected to delay processing, and the arm-region brain wave signal, which has been subjected to filter processing, and updates the filter factor in the filter processing based on the obtained difference. Then, the transfer function obtaining unit 411 repeatedly performs the operation of obtaining the difference between the head-region brain wave signal, which has been subjected to delay processing, and the arm-region brain wave signal, which has been subjected to filter processing, and updating the filter factor; and obtains the filter factor at the time when the difference becomes equal to zero. The filter factor at the time when the difference between the two brain wave signals becomes equal to zero represents the transfer function. In other words, regarding the brain wave signal calculated by multiplying the transfer function to the arm-region brain wave signal (hereinafter, called a corrected head-region brain wave signal) and the head-region brain wave signal having been subjected to delay processing, the difference becomes equal to zero. That is, the corrected head-region brain wave signal can be said to be in antiphase with the head-region brain wave signal having been subjected to delay processing. According to the method explained above, the transfer function obtaining unit 411 calculates the transfer function for correcting the arm-region brain wave signal into the antiphase head-region brain wave signal (the corrected head-region brain wave signal). Meanwhile, the transfer function obtaining unit 411 can obtain the transfer function according to an arbitrary method. For example, the transfer function stored in advance in the memory 30 can be obtained, or the user SU can input the transfer function using the input unit 10.

Although the corrected head-region brain wave signal, which has been calculated, represents the brain wave signal in antiphase with the head-region brain wave signal, it has no difference in the frequency characteristics with respect to the head-region brain wave signal. Hence, by comparing the frequency characteristics of the corrected head-region brain wave signal with the frequency characteristics of the brain wave signal generated at the first body part when the predetermined thought IM arises in the user SU (hereinafter, called a reference brain wave signal), the brain wave correction device 5 can estimate the thought of the user SU from the corrected head-region brain wave signal. More particularly, the brain wave correction device 5 uses the transfer function for correction of the brain wave signal obtained at the second body part and calculates the corrected head-region brain wave signal; and, when the frequency characteristics of the corrected head-region brain wave signal, which has been calculated, are same as the frequency characteristics of the reference brain wave signal, can estimate that the user SU had the predetermined thought IM. Given below is the explanation about the reference brain wave signal that is compared with the corrected head-region brain wave signal.

### (Reference brain wave signal)

The brain wave obtaining unit 412 obtains the reference brain wave signal. The reference brain wave signal is a brain wave signal that is obtained using the first sensor SE1 when the predetermined thought IM arises in the user SU, and that is amplified by the corresponding amplifier AP. In the first embodiment, from among the brain wave signals generated in the user SU when the user SU has the predetermined thought IM (the thought of turning on the light representing the external device ED), the brain wave obtaining unit 412 obtains, as the reference brain wave signal, that signal which is obtained using the first sensor SE1 and which is amplified by the corresponding amplifier AP. Meanwhile, alternatively, a plurality of reference brain wave signals can be obtained. More particularly, for example, the following brain wave signals can be obtained as the reference brain wave signals: a brain wave signal generated when the user SU has a different thought IM2 (a thought of turning off the light representing the external device ED) that is different than the predetermined thought IM; and a brain wave signal generated when the user SU has a thought IM3 that is unrelated to the external device ED. The brain wave obtaining unit 412 stores each obtained reference brain wave signal in the memory 30.

### (Thought estimation operation regarding user SU using brain wave signal obtained at second body part)

Based on the transfer function and the reference brain wave signal explained above, the brain wave correction device 5 estimates the thought of the user SU from the brain wave signal obtained at the second body part. In FIG. 4 is illustrated an explanatory diagram regarding the estimation operation performed by the brain wave correction device 5 for estimating the thought of the user SU from the brain wave signal obtained at the second body part.

### (Brain wave obtaining unit)

The brain wave obtaining unit 412 obtains the brain wave signal at the second body part. In the first embodiment, as illustrated in FIG. 4, the brain wave obtaining unit 412 obtains brain wave signals using the second sensor SE2 attached to an arm region of the user SU. Of the obtained signals, the signals present outside the brain wave signal bandwidth are cut off as noise by the amplifier AP, and only the signals present within the brain wave signal bandwidth are amplified and are used in estimating the thoughts of the user SU.

### (Brain wave correcting unit)

The brain wave correcting unit 413 corrects an arm-region brain wave signal, which is obtained by the brain wave obtaining unit 412 using the second sensor SE2 and which is amplified by the corresponding amplifier AP, to a corrected head-region brain wave signal. More particularly, based on the transfer function, the brain wave correcting unit 413 corrects the arm-region brain wave signal obtained by the transfer function obtaining unit 411, and calculates a corrected head-region brain wave signal that is in antiphase with the head-region brain wave signal and that has the same frequency characteristics as the head-region brain wave signal.

### (Brain wave comparing unit)

The brain wave comparing unit estimates the thought of the user SU based on the corrected head-region brain wave signal, which is calculated by the brain wave correcting unit 413, and the reference brain wave signal obtained in advance at the first body part. More particularly, the brain wave comparing unit 414 compares the corrected head-region brain wave signal with the reference brain wave signal and, when the frequency characteristics of those two brain wave signals are same, estimates that the user SU was having the predetermined thought IM when the reference brain wave signal was obtained. The brain wave comparing unit 414 can compare brain wave signals according to an arbitrary comparison method. For example, the difference between the corrected head-region brain wave signal and the reference brain wave signal can be obtained and, when the difference is equal to or smaller than a predetermined threshold value, can determine that both signals have the same frequency characteristics.

### (Output control unit)

Based on the comparison result about the brain wave signals, the output control unit 415 outputs a control signal to control the external device ED. More particularly, for example, when the comparison result obtained by the brain wave comparing unit 414 about the brain wave signals indicates that the corrected head-region brain wave signal and the reference brain wave signal have the same frequency characteristics, the output control unit 415 outputs a control signal corresponding to the predetermined thought IM that arose in the user SU when the reference brain wave signal was obtained. In the first embodiment, since the predetermined thought IM is set to indicate "turning on the light representing the external device ED", the output control unit 415 outputs a control signal for turning on the light representing the external device ED.

### (Flow of operations)

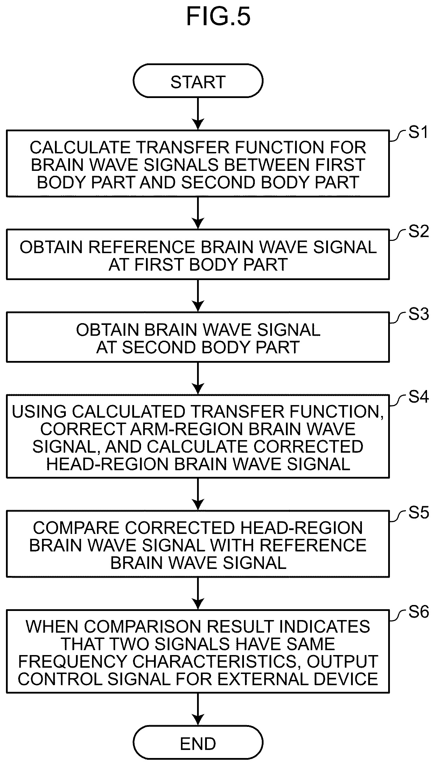

Given below is the explanation about the flow of operations according to the first embodiment. FIG. 5 is a flowchart for explaining the flow of operations performed in the brain wave correction device according to the first embodiment. In the brain wave correction device 5, in the case of estimating the thought arising in the user SU based on the brain wave signal obtained at the second body part, the transfer function obtaining unit 411 obtains brain wave signals, which are generated when the user SU has the predetermined thought IM, using the first sensor SE1 attached to the first body part and the second sensor SE2 attached to the second body part; and amplifies the brain wave signals using the corresponding amplifiers AP. The transfer function obtaining unit 411 performs delay processing with respect to the brain wave signal that is obtained at the first body part and that is amplified by the corresponding amplifier AP (i.e., the head-region brain wave signal); and performs filter processing with respect to the brain wave signal that is obtained at the second body part and that is amplified by the corresponding amplifier AP (i.e., the arm-region brain wave signal). Then, while updating the filter factor for filter processing, the transfer function obtaining unit 411 repeatedly calculates the difference between the head-region brain wave signal, which has been subjected to delay processing, and the arm-region brain wave signal, which has been subjected to filter processing; and calculates, as the transfer function, the filter factor at the time when the difference between the two signals becomes equal to zero (Step S1). The brain wave obtaining unit 412 obtains the brain wave signal that was obtained at the first body part at the time when the user SU had the predetermined thought IM and that is amplified by the corresponding amplifier AP (i.e., obtains the reference brain wave signal) (Step S2). Moreover, the brain wave obtaining unit 412 obtains the brain wave signal that was obtained at the second body part at the time when the user SU had the predetermined thought IM and that is amplified by the corresponding amplifier AP (i.e., obtains the arm-region brain wave signal) (Step S3). The brain wave correcting unit 413 corrects the arm-region brain wave signal, which is obtained by the brain wave obtaining unit 412, based on the transfer function calculated by the transfer function obtaining unit 411; and calculates a corrected head-region brain wave signal (Step S4). The brain wave comparing unit 414 compares the corrected head-region brain wave signal, which is calculated by the brain wave correcting unit 413, with the reference brain wave signal (Step S5). When the comparison result about the brain wave signals indicates that the corrected head-region brain wave signal and the reference brain wave signal have the same frequency characteristics, the output control unit 415 estimates that the user SU had the predetermined thought IM when the reference brain wave signal was obtained, and outputs a control signal corresponding to the predetermined thought IM (Step S6).

### (Effects)

As explained above, in the brain wave correction device 5 according to the first embodiment, brain wave signals generated at the time when the user SU has the predetermined thought IM are obtained using the first sensor SE1 attached to the first body part and using the second sensor SE2 attached to the second body part; the difference between the brain wave signals is obtained; and the transfer function for brain wave signals between the two body parts is calculated. Then, the brain wave signal obtained using the second sensor SE2, which is attached to the second body part, is corrected using the transfer function; and a corrected head-region brain wave signal is calculated that is in antiphase with the head-region brain wave signal and that has the same frequency characteristics as the head-region brain wave signal. Subsequently, the corrected head-region brain wave signal, which has been calculated, is compared with the reference brain wave signal obtained in advance at the first body part, and the thought arising in the user SU is estimated. According to the application concerned, brain wave signals having high accuracy can be obtained at body parts other than the head region, and the thoughts arising in the user SU can be estimated.

### (Second embodiment)

Given below is the description of a second embodiment. In the first embodiment, a thought arising in the user SU is estimated from the brain wave signal obtained by a single brain wave sensor attached at the second body part. The second embodiment differs from the first embodiment in the way that a thought arising in the user SU is estimated from the brain wave signals obtained by the brain wave sensors SE2 attached to a plurality of second body parts. In the second embodiment, the configuration identical to the first embodiment is not explained again.

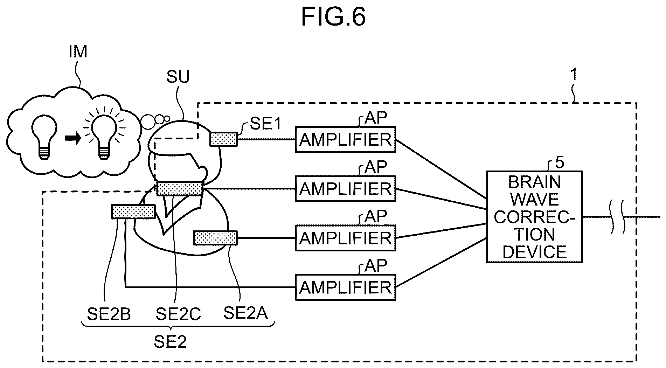

FIG. 6 is a schematic diagram of the brain wave measurement device according to the second embodiment. As illustrated in FIG. 6, the brain wave measurement device 1 according to the second embodiment includes the brain wave correction device 5; the first sensor SE1; a plurality of second sensors SE2; and the amplifiers AP that connect the first sensor SE1 and the second sensors SE2 to the brain wave measurement device 1. According to the second embodiment, the first sensor SE1 is attached to the head region representing the first body part. The second sensors SE2 are attached to second body parts (the body parts other than the head region). It is desirable that the second sensors SE2 are attached to mutually different body parts. In the example illustrated in FIG. 6, second sensors SE2A, SE2B, and SE2C are disposed as the second sensors SE2. More particularly, the second sensor SE2A is attached to one arm region, the second sensor SE2B is attached to the other arm region on the opposite side of the second sensor SE2A, and the second sensor SE2C is attached to the neck region. However, the number of second sensors SE2 is not limited to three, and there can be an arbitrary number of second sensors SE2. Moreover, the body parts to which the second sensors SE2 are attached can be decided in an arbitrary manner.

Based on the brain wave signal obtained at the first body part and the brain wave signals obtained at the second body parts, the transfer function obtaining unit 411 obtains a transfer function for brain wave signals between the first body part and each of the second body parts. More particularly, based on the first sensor SE1 attached to the first body part, the second sensor SE2A attached to one arm region, the second sensor SE2B attached to the other arm region on the opposite side of the second sensor SE2A, and the second sensor SE2C attached to the neck region; the transfer function obtaining unit 411 obtains the brain wave signals at the time when the user SU has the predetermined thought IM, and calculates a transfer function for brain wave signals between the first body part and each of the second body parts. The predetermined thought IM at that time can be set in an arbitrary manner. For example, the predetermined thought IM can be meant for operating the external device ED, or can be a thought unrelated to the operation of the external device ED. The method for calculating the transfer function is identical to the method according to the first embodiment. Hence, that explanation is not given again.

The brain wave obtaining unit 412 obtains the brain wave signals at the second body parts. In the second embodiment, as illustrated in FIG. 6, the brain wave obtaining unit 412 obtains the brain wave signals using the second sensor SE2A attached to one arm region of the user SU, the second sensor SE2B attached to the other arm region on the opposite side of the second sensor SE2A, and the second sensor SE2C attached to the neck region of the user SU. Of the obtained signals, the signals present outside the brain wave signal bandwidth are cut off as noise by the corresponding amplifiers AP, and only the signals present within the brain wave signal bandwidth are amplified and are used in estimating the thought of the user SU.

The brain wave correcting unit 413 makes use of the transfer functions, which are calculated by the transfer function obtaining unit 411, to correct the brain wave signals, which are obtained at the second body parts by the brain wave obtaining unit 412 using the second sensors SE2A to SE2C and which are amplified using the corresponding amplifiers AP; synthesizes the corrected brain wave signals; and calculates a corrected head-region brain wave signal according to the second embodiment. More particularly, the brain wave correcting unit 413 adds the three brain wave signals that are obtained at the second body parts and that are corrected using the corresponding transfer functions. At that time, with respect to the three brain wave signals that are obtained at the second body parts and that are corrected using the corresponding transfer functions, the brain wave correcting unit 413 can perform filter processing in such a way that the frequency band in the range between 0 Hz and 30 Hz is achieved, and then can add the three brain wave signals. Herein, the frequency band extracted in filter processing is not limited to the range between 0 Hz to 30 Hz, and it serves the purpose as long as the frequency band of the brain wave signal detected by the first sensor SE1 matches with the frequency band of the brain wave signals detected by the second sensors SE2. As compared to an individual corrected head-region brain wave signal that is obtained at a single second part instead of a plurality of second parts and that is corrected by the corresponding transfer function, the corrected head-region brain wave signal generated as a result of the addition has a greater amplitude and exhibits more prominent frequency characteristics. In other words, as a result of adding the three brain wave signals that have been corrected by the corresponding transfer functions, it becomes possible to calculate a corrected head-region brain wave signal having higher accuracy (i.e., having the frequency characteristics more similar to the frequency characteristics of the brain wave signal obtained at the first part) as compared to an individual corrected head-region brain wave signal. Meanwhile, the subsequent operations performed by the brain wave comparing unit 414 and the output control unit 415 are identical to the first embodiment. Hence, that explanation is not given again.

### (Effects)

As explained above, in the brain wave correction device according to the second embodiment, the brain wave signals generated at the time when the user SU has the predetermined thought IM are obtained using the first sensor SE1 attached to the head region, the second sensor SE2A attached to one arm region, the second sensor SE2B attached to the other arm region on the opposite side of the second sensor SE2A, and the second sensor SE2C attached to the neck region. Then, from the distance between the brain wave signal obtained at the first body part and the brain wave signal obtained at each of the second body parts, a transfer function is calculated for brain wave signals between body parts. Then, the brain wave signal obtained at each of the second body parts is corrected using the corresponding transfer function. The three corrected brain wave signals are added to calculate a corrected head-region brain wave signal that is in antiphase with and has the same frequency characteristics to the brain wave signal obtained at the first body part. Subsequently, the corrected head-region brain wave signal, which has been calculated, is compared with the reference brain wave signal obtained in advance at the first body part, and the thought arising in the user SU is estimated. According to the application concerned, based on the brain wave signals calculated at a plurality of second body parts, it becomes possible to obtain brain wave signals having high accuracy and accordingly estimate the thought arising in the user SU.

Moreover, since the corrected head-region brain wave signal according to the second embodiment is generated as a result of adding three brain wave signals that are obtained at a plurality of second body parts and that are corrected using the corresponding transfer functions, it becomes possible to obtain a brain wave signal having higher accuracy (i.e., having the frequency characteristics more similar to the frequency characteristics of the brain wave signal obtained at the first part) as compared to the corrected head-region brain wave signal obtained according to the first embodiment.

### (Effects)

As explained above, the brain wave correction device 5 according to the application concerned includes: the transfer function obtaining unit 411 that, based on the brain wave signal obtained at the head region representing the first body part and the brain wave signal obtained at the second body part different than the first body part at the time when the user SU has the predetermined thought IM, obtains a transfer function for brain wave signals between body parts; the brain wave obtaining unit 412 that obtains the brain wave signal at the second body part; the brain wave correcting unit 413 that makes use of the transfer function to correct the brain wave signal obtained at the second body part, and calculates a corrected head-region brain wave signal; and the brain wave comparing unit 414 that, based on the corrected head-region brain wave signal and based on the reference brain wave signal obtained in advance at the first body part, estimates the thought arising in the user SU. According to the application concerned, brain wave signals having high accuracy can be obtained at a body part other than the head region.

Moreover, according to the application concerned, the transfer function obtaining unit 411 calculates a transfer function based on the brain wave signal obtained at the first body part and the brain wave signal obtained at the second body part at the time when the user SU has the predetermined thought IM. Thus, according to the application concerned, the transfer function can be calculated based on the brain wave signal obtained at the first body part and the brain wave signal obtained at the second body part.

Furthermore, according to the application concerned, the transfer function obtaining unit 411 calculates the delay period for the brain wave signal obtained at the second body part with respect to the brain wave signal obtained at the first body part; corrects the brain wave signal at the first body part based on the delay period; and, based on the post-correction brain wave signal at the first body part and the brain wave signal at the second body part, calculates the transfer function for brain wave signals between body parts. According to the application concerned, based on the brain wave signal obtained at the first body part and the brain wave signal obtained at the second body part, the transfer function can be calculated by taking into account the brain wave acquisition timings at (the delay period between) the first body part and the second body part.

Moreover, according to the application concerned, the transfer function obtaining unit 411 calculates transfer functions based on the brain wave signal obtained at the first body part and the brain wave signals obtained at a plurality of second body parts at the time when the user SU has the predetermined thought IM; and the brain wave correcting unit 413 corrects the brain wave signal at each of the second body part using the corresponding transfer function, synthesizes the corrected brain wave signals, and calculates a corrected head-region brain wave signal. Thus, according to the application concerned, the brain wave signals at the second body parts are corrected using the transfer functions, and the corrected brain wave signals are synthesized to calculate a corrected head-region brain wave signal. As a result, it becomes possible to calculate a corrected head-region brain wave signal having higher accuracy.

A brain wave correction method according to the application concerned includes: an obtaining step that, based on the brain wave signal obtained at the head region representing the first body part and the brain wave signal obtained at the second body part different than the first body part at the time when the user SU has the predetermined thought IM, includes obtaining a transfer function for brain wave signals between body parts; an obtaining step for obtaining the brain wave signal at the second body part; a correcting step for correcting the brain wave signal, which is obtained at the second body part, based on the transfer function and calculating a corrected head-region brain wave signal; and an estimating step that, based on the corrected head-region brain wave signal and based on the reference brain wave signal obtained in advance at the first body part, includes estimating the thought arising in the user SU. According to the application concerned, at a body part other than the head region, it becomes possible to obtain brain wave signals having high accuracy.

The program according to the application concerned causes a computer to execute: an obtaining step that, based on the brain wave signal obtained at the head region representing the first body part and the brain wave signal obtained at a second body part different than the first body part at the time when the user SU has the predetermined thought IM, includes obtaining a transfer function for brain wave signals between body parts; an obtaining step for obtaining the brain wave signal at the second body part; a correcting step for correcting the brain wave signal, which is obtained at the second body part, based on the transfer function and calculating a corrected head-region brain wave signal; and an estimating step that, based on the corrected head-region brain wave signal and based on the reference brain wave signal obtained in advance at the first body part, includes estimating the thought arising in the user SU. According to the application concerned, at a body part other than the head region, it becomes possible to obtain brain wave signals having high accuracy.

Till now, the embodiments of the present invention were described. However, the present invention is not limited by the details given in the embodiments. Moreover, the constituent elements described above are not to be thus limited but are to be construed as embodying all modifications and alternative constructions that may occur to one skilled in the art that fairly fall within the basic teaching herein set forth.

### Industrial Applicability

The brain wave correction device and the brain wave correction method can be used, for example, as a brain wave sensor or as a brain wave switch that controls external devices.

This application is based upon and claims the benefit of priority from Japanese Patent Application No. 2023-089548, filed on May 31, 2023; the entire contents of which are incorporated herein by reference.

### Reference Signs List

- 1: brain wave measurement device
- 5: brain wave correction device
- SU: user
- ED: external device
- IF: external device interface
- IM: predetermined thought
- AP: amplifier
- SE1: first sensor
- SE2: second sensor

## Claims

1. A brain wave correction device comprising:
a transfer function obtaining unit that, based on a brain wave signal at a first body part and a brain wave signal at a second body part different than the first body part at time when a user has a predetermined thought, obtains a transfer function for brain wave signals between the body parts;
a brain wave obtaining unit that obtains a brain wave signal at the second body part;
a brain wave correcting unit that calculates a corrected head-region brain wave signal which is a brain wave signal obtained by correcting, based on the transfer function, the brain wave signal obtained at the second body part; and
a brain wave comparing unit that, based on the corrected head-region brain wave signal and based on a reference brain wave signal obtained in advance at the first body part, estimates thought arising in the user.

2. The brain wave correction device according to claim 1, wherein the transfer function obtaining unit calculates the transfer function based on the brain wave signal at the first body part and the brain wave signal at the second body part at time when the user has the predetermined thought.

3. The brain wave correction device according to claim 2, wherein the transfer function obtaining unit
calculates a delay period for the brain wave signal at the second body part with respect to the brain wave signal at the first body part,
performs correction of the brain wave signal at the first body part based on the delay period, and
calculates, based on the brain wave signal at the first body part after the correction and based on the brain wave signal at the second body part, the transfer function for the brain wave signals between the body parts.

4. The brain wave correction device according to any one of claims 1 to 3, wherein
based on the brain wave signal at the first body part and based on the brain wave signal at each of a plurality of the second body part at time when the user has the predetermined thought, the transfer function obtaining unit calculates the transfer function for each of the brain wave signals, and
the brain wave correcting unit
corrects, based on each of the calculated transfer functions, the brain wave signal obtained at each of the plurality of second body parts,
synthesizes the corrected brain wave signals, and
calculates the corrected head-region brain wave signal.

5. A brain wave correction method comprising:
an obtaining step that, based on a brain wave signal at a first body part and a brain wave signal at a second body part different than the first body part at time when a user has a predetermined thought, includes obtaining a transfer function for brain wave signals between the body parts;
an obtaining step for obtaining a brain wave signal at the second body part;
a calculating step that includes correcting, based on the transfer function, the brain wave signal obtained at the second body part and calculating a corrected head-region brain wave signal; and
an estimating step that, based on the corrected head-region brain wave signal and based on a reference brain wave signal obtained in advance at the first body part, includes estimating thought arising in the user.
